# EUROPEAN PATENT APPLICATION

(11) **EP 3 243 544 A1**
(43) Date of publication of application: **15.11.2017**
(21) Application number: 15876727.7
(22) Date of filing: 23.12.2015
(51) Int. Cl.: A61M 16/00

(54) **IMPROVEMENTS TO RESUSCITATION DEVICE FOR VICTIMS OF CARDIORESPIRATORY ARRESTS**

(30) Priority: 05.01.2015 ES 201530007
(71) Applicant: Maselli, Javier Ernesto, 08020 Barcelona (ES)
(72) Inventor: Maselli, Javier Ernesto, 08020 Barcelona (ES)
(74) Representative: Herrero & Asociados, S.L.
(86) International application number: PCT/ES2015/070947
(87) International publication number: WO 2016/110605

(57) **Abstract**

The resuscitation device comprises a curved tube (3) which is inserted into the mouth and the throat of the patient, a straight segment (3b) remaining on the outside, where two one-way safety valves (4, 5, 7, 8, 10) are arranged, for preventing the return of fluid, said device (1) also including a prophylactic mouthpiece (2) used by the resuscitating person in order to blow the air, and a protective mask (20) which covers the mouth and the nose of the resuscitator, the mouthpiece (2) and mask (20) assembly being able to rotate in both directions so as to adopt different positions. The improvements are characterized in that the tube (3) is mounted on the rest of the device (1) in a pressurized detachable manner.

It enables the tube to be mounted in its position of use in a very simple manner, as it only requires the pressurized coupling of the end of the tube in the corresponding housing thereof.

## Description

The present invention relates to improvements to the main patent P201101183 for "resuscitation device", which was designed and made to obtain various and notable advantages to perform the cardiopulmonary resuscitation maneuver (CPR) with complete prophylaxis.

### Background of the invention

To date, if we speak of prophylaxis, only one aid element for the maneuver is known (a mask similar to that used by the anesthetist). It can cause injury to the lips, since it must be pressed so that the air enters the mouth. There is also a mask attached to a bag (the function of which is to blow air). In this case, the intervention of a second person who helps the resuscitator hold this apparatus is needed.

Lastly, we can mention some type of tube or cannula that is used (and has been used for centuries) in surgery or with patients admitted to medical centers. This makes it possible to intubate the person without any type of protection or prophylaxis for the resuscitator. It is simply a tube (it does not prevent infection).

With the instruments of the state of the art, the resuscitator is exposed to direct contact and all the risks it entails. The patient is exposed to possible choking (on their own fluid, their tongue), to self-injury due to biting, injuries from masks, and to the most serious: delays and/or failures in the maneuver, possibly leading to severe, irreversible brain damage and even death. All of this is caused by failing to have a single, more practical, quicker and more efficient tool that is perfectly suitable for the maneuver.

### Description of the invention

The present invention provides a device that resolves the aforementioned drawbacks and has other advantages which are described below.

The device is a CPR resuscitation device for topical, oropharyngeal or supraglottic use which by being the type made up of one or more parts, according to its manufacture, is characterized by the fact that its configuration is by way of a curved tube which is inserted into the mouth and the throat of the patient, a straight segment remaining on the outside, where two one-way safety valves are arranged, for preventing the return of fluid, said device also including a prophylactic mouthpiece used by the resuscitating person in order to blow the air, and a protective mask which covers the mouth and the nose of the resuscitator, the mouthpiece and mask assembly being able to rotate in both directions so as to adopt different positions.

The device has the advantage that it prevents infection and includes in a single instrument the possibility of performing several actions that streamline the intervention of the resuscitator, earning valuable, precious time from the start of the CPR maneuver, with complete prophylaxis. Furthermore, one resuscitator can use it easily, since it remains inserted into the mouth of the patient while the resuscitator performs the cardiac massages.

Preferably, said curved tube is inserted until the pharynx and includes the limiting intake edge that acts as a stop against the teeth/gums of the patient.

Advantageously, said two one-way safety valves are configured to prevent the breath and all types of secretions in the mouth of the patient from coming in contact with the resuscitator, including the outside of the oropharyngeal tube, two side outlet holes to enable the fluids of the patient to be exhaled.

Furthermore and advantageously, said mouthpiece has two side fastening reinforcements for the mouth of the resuscitator.

Preferably, the device comprises a second protective mask for the victim that covers their mouth or their entire face and is fixed or detachable.

The device is provided so that, in a quick and practical way, (thanks to an ergonomic tube or cannula) the mouth and pharynx of the patient are freed to let air pass through, the tongue is retained to prevent choking (for example, caused by a lax tongue) or injury in the case of biting. All this takes place quickly during the time in which air is taken in and exhaled and cardiac massage for resuscitation is carried out.

Furthermore, thanks to the mouthpiece with the mask it has and the one-way valves thereof, contact of the resuscitator with the mouth of the patient is impeded, thus preventing infection through the saliva, breath, vomit or blood. HIV, SARS, hepatitis, infections and all types of illnesses (more than 20 different fatal or very serious illnesses).

According to the improvements of the present invention, the tube is mounted on the rest of the device in a pressurized detachable manner.

According to a preferred embodiment, for said mounting in a pressurized detachable manner, the tube comprises elastic protrusions that are housed in complementary holes in the position of use of the tube. For example, the elastic protrusions are elongated and the complementary holes are rectangular.

In this way, mounting the tube in its position of use is very simple, as it only requires the pressurized coupling of the end of the tube in the corresponding housing thereof, the protrusions being housed in the complementary holes thanks to their elastic nature. To remove the tube, it is simply necessary to press said protrusions towards the inside of the tube.

### Brief description of the drawings

For the purpose of helping to make the foregoing description more readily understandable, it is accompanied by a set of drawings which, schematically and by way of illustration and not limitation, represent an embodiment.
Figure 1 is a perspective view of the device according to the main patent.
Figure 2 is a longitudinal cross section of the device according to the main patent.
Figure 3 is a perspective view that shows the oropharyngeal tube with the side outlet holes and the end for inserting the mouthpiece assembly of the device according to the main patent.
Figure 4 is a perspective view that shows the part that constitutes the body of the ergonomic mouthpiece of the device according to the main patent.
Figure 5 is a perspective view that shows the one-way piston valve of the device according to the main patent.
Figure 6 is an elevation view of the device according to the present invention, with the tube separated from the rest of the device.
Figure 7 is an elevation view of the device according to the present invention, with the tube coupled in its position of use.
Figure 8 is an elevation view of the device according to the present invention, with a plurality of tubes that can be coupled to the rest of the device.

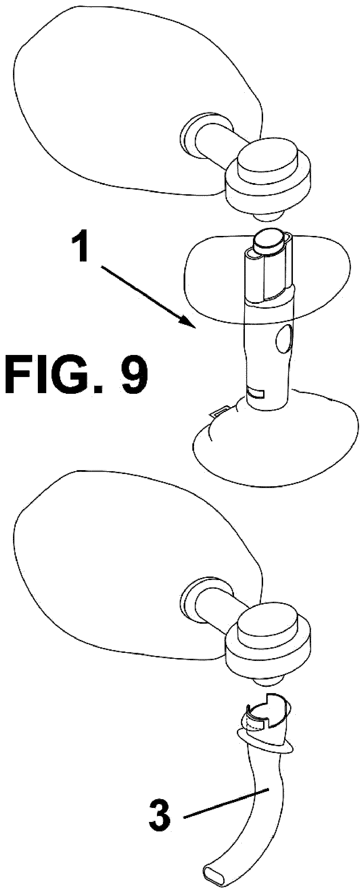

Figure 9 is a view of the device according to the present invention, in which it can be seen that the tube and the rest of the device can also be used separately.

### Description of a preferred embodiment

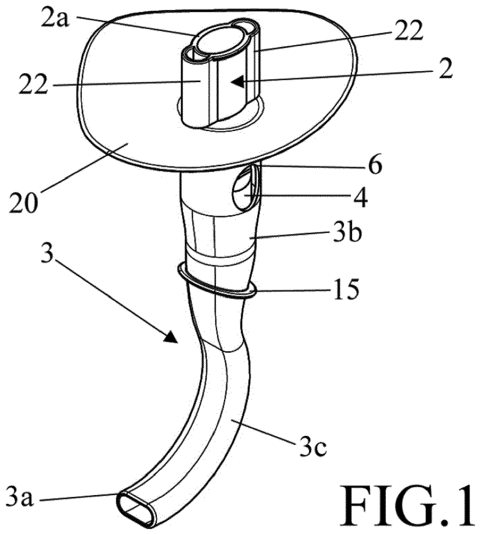

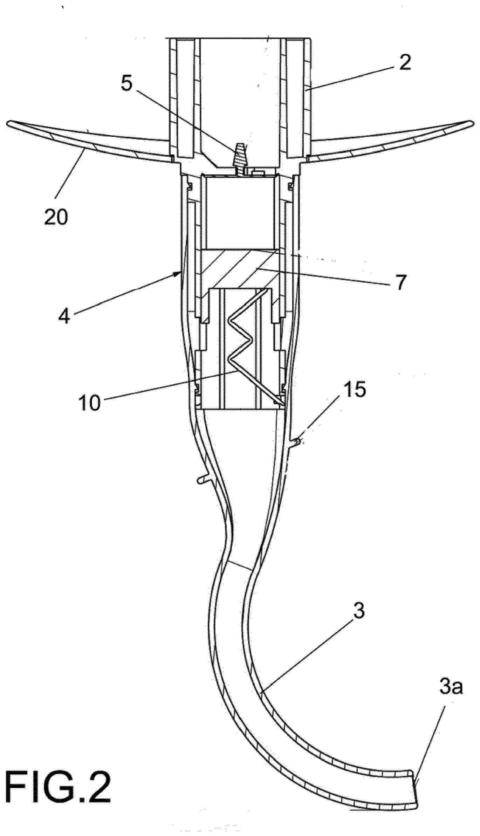

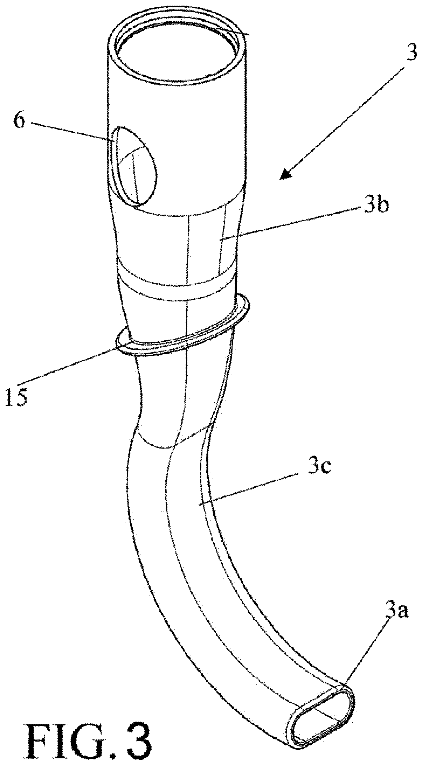

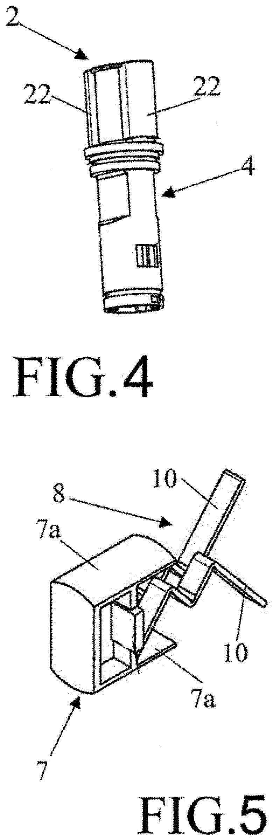

To provide a complete description of the device, Figures 1 to 5 show the device according to the main patent, the description of which is also included in the present specification.

The device 1 has a structure that is solid enough to be inserted into the mouth and throat of the patient and at the same time has the flexibility needed to not create injuries. It can be lubricated. It is made from a plastic material internationally approved for its use in this area. It is translucent and makes it possible to see the existence of fluid or if the patient is bleeding or vomiting.

The end 3a, 3c that enters the mouth of the patient is curved and ergonomic, with a limiting intake stop or edge 15 in the mouth (in contact with the teeth and/or gums). The opposite external end 3b that the resuscitator uses has a mouthpiece 2 that enables fastening using the teeth and a mask 20 that acts as a barrier and covers the lips and nose of the resuscitator (it is designed for the case of all types of splatter).

There are two non-return valves, the main valve in the form of a piston 7, 7a that rises and falls (enabling the opening to let the blown air pass through and closing with exhalation, which has an outlet to the exterior). The secondary valve, in the form of a flexible barrier 5, also enables the air blown by the resuscitator to pass through, preventing the flow in the opposite direction, reinforcing the security of the first valve.

The outlet of the air and possible fluids of the patient is towards the exterior, through two outlets or side holes 6 of the tube body 3.

The resuscitator will then use the mouthpiece 2, on the outer end 2a of the device 1, which has two side reinforcements 22 so that it can be held with their own teeth. The protection or mask 20 that completely covers the lips and nose, as well as the distance of separation between the mouths created by the tube 3 of the device 1, prevents any contact with the patient, their mouth, skin, saliva and other fluids, thus preventing the possible spread of infections, HIV, fevers, viruses, etc. It therefore makes the optimal performance of the CPR maneuver possible. The victim can also have a protective mask that covers their lips.

The resuscitator must introduce the curved body of the oropharyngeal device 1 into the mouth of the patient, thus ensuring that their mouth remains open, enabling air to pass through. The tubed device 1 holds the tongue, preventing choking and enabling the air and fluids to circulate. It also prevents biting by protecting the tongue and the rest of the mouth.

The main valve 4 operates as a piston 7 that remains in a closed position. It is opened when the resuscitator blows. Thanks to a spring 8 with legs 10 in the form of a zig-zag, it returns to its resting position (closed). The one-way valves 4, 5 prevent the breath and fluids exhaled by the patient from coming in contact with the mouth of the resuscitator. The second valve is flexible and also one-way. It is located inside the body of the device 1 and opens when the resuscitator blows. In the case of exhalation, these valves close, leaving the outlet open, which always opens towards the outside of the device, without contact.

The assembled set of parts that comprise the instrument is pressurized. The mouthpiece 2 and the protection mask 20 or barrier thereof rotate in both directions (enabling its use in different positions, thus providing an additional advantage). It is sterilized in units, and provided in an easy-open package.

In the case of using a resuscitation bag, the device is pressurized and quickly placed in the mouthpiece 2 of the respirator on one side and in the bag on the other side. In this way, the resuscitator "blows" with the bag and a second resuscitator is not needed.

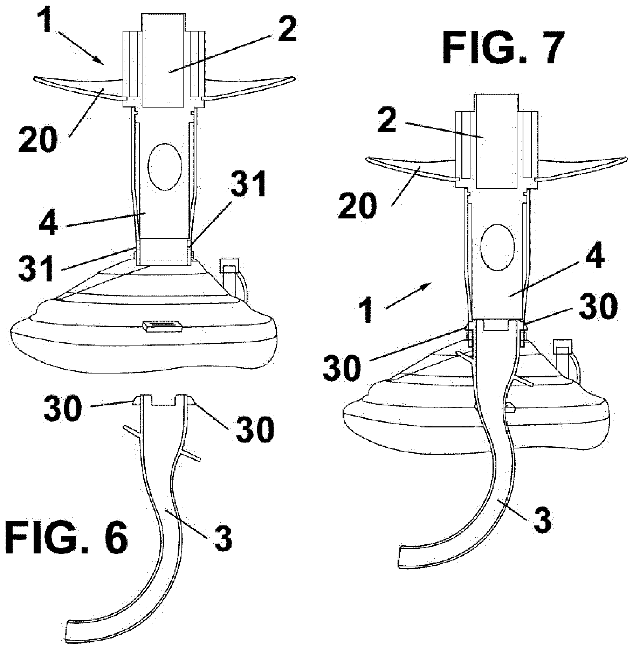

In Figures 6 and 7, the resuscitation device with the improvements object of the present invention is shown. For reasons of simplicity, in these figures, the same numerical references are used as in the preceding embodiment to indicate the same or similar elements.

According to the improvements of the present invention, the tube 3 is mounted on the rest of the device 1 in a pressurized detachable manner.

According to the embodiment shown, the tube 3 comprises elastic protrusions 30 that are housed in complementary holes 31 in the position of use of the tube 3. For example, the elastic protrusions 30 are elongated and the complementary holes 31 are rectangular.

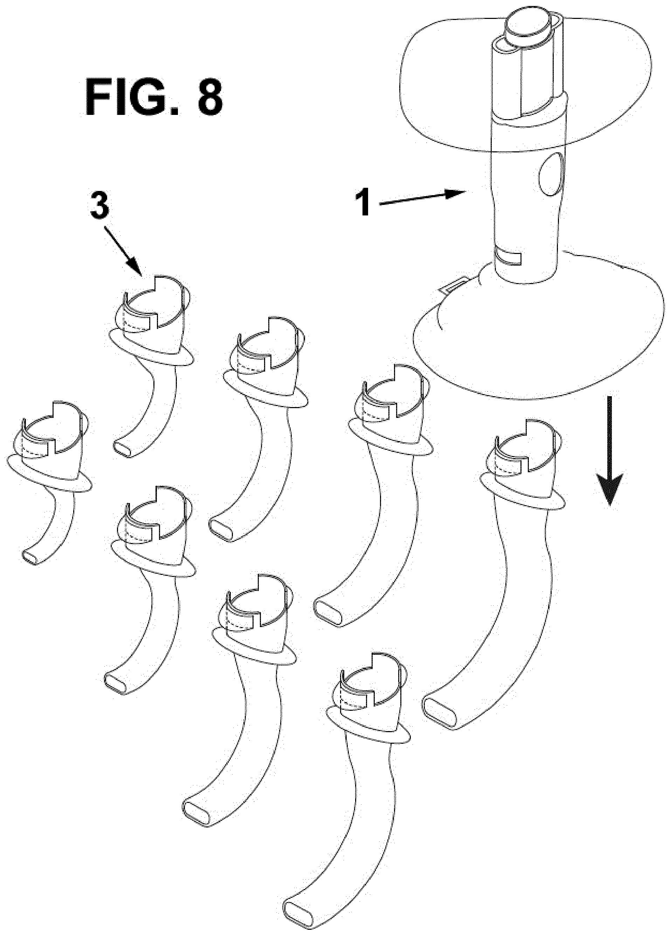

The resuscitation device with the improvements according to the present invention enables the different cannula or tube sizes to be exchanged at the will of the user (for example, eight different sizes, from neonatal pediatric to adult), as shown in Figure 8.

The cannulas or tubes 3 are placed and removed in a pressurized detachable manner, with sufficient robustness to be able to handle the device and exert force, without affecting the structure thereof.

The tube 3 is easily removed with two fingers, making it possible to replace one size of the tube 3 with another of a different size, which is once again placed in a pressurized manner, using the same device.

If so desired, the device can also be used without the cannula or tube for the user who does not want to touch or open the mouth of the patient.

The device according to the present invention also has the advantage of being able to connect standard apparatuses to the tube or cannula, which remains inserted into the mouth of the patient once the rest of the device is removed, such as, for example, the same emergency ventilation device, an oxygen line, as well as the input of endotracheal tubes, gastric tubes, etc. This is noteworthy because other cannulas of this type do not enable different standard devices to be coupled to them.

Despite the fact that reference has been made to a specific embodiment of the invention, it is evident for a person skilled in the art that numerous variations and changes may be made to the device described, and that all the aforementioned details may be substituted by other technically equivalent ones, without detracting from the scope of protection defined by the attached claims.

## Claims

1. Improvements to the resuscitation device for victims of cardiorespiratory arrests, which comprises a curved tube (3) which is inserted into the mouth and the throat of the patient, a straight segment (3b) remaining on the outside, where two one-way safety valves (4, 5, 7, 8, 10) are arranged, for preventing the return of fluid, said device (1) also including a prophylactic mouthpiece (2) used by the resuscitating person in order to blow the air, and a protective mask (20) which covers the mouth and the nose of the resuscitator, the mouthpiece (2) and mask (20) assembly being able to rotate in both directions so as to adopt different positions, **characterized in that** the tube (3) is mounted on the rest of the device (1) in a pressurized detachable manner.

2. The improvements according to claim 1, **characterized in that** the tube (3) comprises elastic protrusions (30) that are housed in complementary holes (31) in the position of use of the tube (3).

3. The improvements according to claim 2, **characterized in that** the elastic protrusions (30) are elongated and the complementary holes (31) are rectangular.
